# EUROPÄISCHE PATENTSCHRIFT

(11) **EP 2 596 873 B1**
(45) Veröffentlichungstag und Bekanntmachung des Hinweises auf die Patenterteilung: **03.05.2017**
(21) Anmeldenummer: 12007468.7
(22) Anmeldetag: 02.11.2012
(51) Int. Cl.: B05C 17/005

(54) **Mehrkomponenten-Kartuschensystem mit verschiebbaren Verschlüssen in den Kartuschen**
Multiple component cartridge system with sliding locks in the cartridges
Système de cartouche à plusieurs composants avec fermetures mobiles dans les cartouches

(30) Priorität: 25.11.2011 DE 102011119357
(43) Veröffentlichungstag der Anmeldung: 29.05.2013
(73) Patentinhaber: Heraeus Medical GmbH, 61273 Wehrheim (DE)
(72) Erfinder: Vogt, Sebastian, 99092 Erfurt (DE); Schnieber, Tim, 60439 Frankfurt (DE)
(74) Vertreter: Schultheiss & Sterzel Patentanwälte PartG mbB

(56) Entgegenhaltungen:
- EP-A1- 1 728 560
- EP-A2- 1 430 959
- WO-A2-2005/016170
- WO-A2-2007/104037
- DE-A1-102005 041 961
- DE-B3-102005 041 962

## Beschreibung

Die Erfindung betrifft Kartuschensysteme zum Mischen und Applizieren eines Mischguts, insbesondere eines medizinischen Zements, umfassend zumindest zwei parallel zueinander angeordnete Kartuschen und einen Mischraum mit einer Auslassöffnung, wobei die Kartuschen jeweils zumindest eine Öffnung in den Kartuschenwänden umfassen, die die Kartuschen mit dem Mischraum verbinden, die Kartuschen jeweils einen Förderkolben zum Austreiben von Ausgangskomponenten des Mischguts aus den Kartuschen durch die Öffnungen umfassen und jeder Kartusche zumindest ein in Längsrichtung der Kartuschen verschiebbarer Verschluss zugeordnet ist, wobei die Verschlüsse die Öffnungen der Kartuschen in einer Ausgangsposition verschließen und die Öffnungen in einer Endposition der verschiebbaren Verschlüsse zumindest bereichsweise geöffnet sind.

Kartuschensysteme zum Mischen und Applizieren eines Mischguts können aus mehreren Komponenten bestehen und sollen eine sichere Aufbewahrung und einen sicheren Verschluss für Komponenten in zumindest zwei Kartuschen vor ihrer Verwendung gewährleisten. Unmittelbar vor der Applikation des Mischguts soll das Kartuschensystem sicher und schnell zu öffnen sein, wobei eine synchrone Öffnung der einzelnen Kartuschen erstrebenswert ist.

Reaktive pastöse Zwei- oder Mehrkomponentensyteme müssen nach ihrer Herstellung bis zur Applikation getrennt aufbewahrt werden, um vorzeitige, unbeabsichtigte Reaktionen der Komponenten zu verhindern. Kartuschensysteme für die Applikation von pastösen Zwei- oder Mehrkomponentensystemen sind seit Jahrzehnten bekannt. Beispielhaft seien dafür die Dokumente CH 669 164 A5, EP 0 607 102 A1, EP 0 236 129 A2, DE 3 440 893 A1, US 4,690,306 A, US 2009/062808 A1, EP 0 787 535 A1, WO 2006/005 206 A1, EP 0 693 437 A1, EP 0 294 672 A, EP 0 261 466 A1 und EP 2 008 707 A1 genannt. Nach Befüllen der Kartuschen mit reaktiven Pasten müssen diese sicher bis zur Applikation verschlossen bleiben. Die Vermischung der pastösen Zwei- oder Mehrkomponentensysteme erfolgt unmittelbar bei der Applikation üblicherweise mit Hilfe von statischen Mischern. Exemplarisch seien dafür die Dokumente GB 1,188,516 A, US 2,125,245 A, US 5,968,018 A, US 4,068,830 A, US 2003/179648 A1, EP 1 799 335 A1, EP 0 664 153 A1 und EP 0 289 882 A1 angeführt. Bewegliche Kolben, die auch zum Austragen des Kartuscheninhalts dienen, dichten dabei üblicherweise die Kartuschenböden ab.

Zum Verschließen des Kartuschensystems wurde eine Reihe von Lösungen vorgeschlagen.

Ein einfaches, aber sehr wirksames Prinzip besteht darin, das Kartuschensystem mit einem drehbaren Verschluss zu verschließen (EP 0 431 347 A1, DE 2 017 292 A1, US 3,215,298 A). Der Verschluss wird vor der Applikation herausgedreht. Anschließend wird ein Austragsrohr in ein Gewinde am Kartuschensystem eingedreht oder mit einem, ein Gewinde nachbildendem Zapfensystem fixiert. Nachteilig ist hieran, dass der Anwender zweimal Drehbewegungen ausführen muss, bis das Pastenmaterial ausgetrieben werden kann. Weiterhin kann es passieren, dass der Verschluss herausgedreht wird und das Austragsrohr erst später aufgesetzt wird. Zwischen dem Öffnen der Kartuschen und dem Einsetzen des Austragsrohrs kann es, insbesondere wenn flüchtige Substanzen in den Pasten enthalten sind, zur Verdunstung von Bestandteilen der Pasten kommen.

Der gegenwärtig sehr häufig, insbesondere in der Klebstoff- und Dichtungsmittelindustrie verwendete Verschluss basiert darauf, dass am Kartuschenkopf das Wandmaterial der Kartusche sehr dünn ausgebildet ist, so dass diese Wandung leicht durchstochen werden kann. Beim Durchstoßen werden Partikel aus der Wandung abgelöst, die dadurch in das pastöse Material gelangen.

Die Rückseite der Kartuschen wird üblicherweise durch bewegliche Kolben verschlossen, die zum Austreiben der Pasten bei der Applikation bestimmt sind. Bei feuchtigkeitsund luftempfindlichen Pasten können Aluminiumkartuschen verwendet werden, die mit Kunststoffkolben verschlossen sind und über die zur Abdichtung Aluminiumzylinder, die an einer Seite geschlossen sind, eingepresst werden. Bei der Applikation der Pasten wird der einseitig geschlossene Aluminiumzylinder zusammen mit dem Kolben mit Hilfe von Auspresspistolen nach vorne in Richtung Kartuschenkopf bewegt und die Paste dabei ausgetrieben. Bei medizinischen Anwendungen kann jedoch ein Kontakt von Pasten mit Aluminiumoberflächen problematisch sein.

Ein Kartuschensystem basiert auf der Verpackung von pastösen Mehrkomponentensystemen in Schlauchbeuteln (WO 2010/006455 A1). Die versiegelten Schlauchbeutel werden dabei in Kartuschen eingebracht. Schlauchbeutel haben den Vorteil, dass sie zur Verpackung von Pasten geeignet sind, die flüchtige Bestandteile enthalten. Schlauchbeutel aus Verbundmaterialien, wie zum Beispiel Aluminiumverbundbeutel, sind dafür besonders geeignet. Die Öffnung der Schlauchbeutel erfolgt durch Schneiden, die sich beim Eindrehen des Austragsrohrs mit drehen. Bei der Drehbewegung der Schneiden werden die Beutel aufgeschnitten und so Öffnungen in den Kartuschen zum Austragen des Inhalts bereitgestellt. Der pastöse Beutelinhalt wird anschließend durch diese Öffnungen in den Kartuschen in Richtung des statischen Mischers ausgepresst.

Nachteilig ist hieran, dass die Verpackung von pastösen Materialien in Schlauchbeuteln und zusätzlich in Kartuschen sehr kostenintensiv und nur speziellen Anwendungen vorbehalten ist. Für viele Anwendungen, vor allem im medizinischen Bereich, ist es zudem Problematisch, dass sich Teile der geschnittenen Schlauchbeutel ablösen können und dann in die pastösen Komponenten gelangen und so das Mischgut kontaminieren können.

Bei der Verwendung von Kartuschensystemen für sterile pastöse Medizinprodukte ist es notwendig, dass nicht nur die Pasten sondern natürlich auch die Kartuschen und das sekundäre Packmittel in steriler Form dem medizinischen Anwender zur Verfügung gestellt werden. So können zum Beispiel nach aseptischer Befüllung der zuvor sterilisierten Kartuschen diese direkt in ein steriles Packmittel überführt werden. Weiterhin kann es bei bestimmten Produkten sinnvoll sein, die Oberflächen der befüllten Kartuschen zusammen mit den Packmitteln nach erfolgter Verpackung zu sterilisieren. Neben der Gammasterilisation, die bei polymerisierbaren Pastensystemen nicht angewendet werden kann, besteht die Möglichkeit mit dem Gas Ethylenoxid zu sterilisieren.

Problematisch bei Pastensystemen, die Monomere mit hohem Dampfdruck enthalten, ist bei einer solchen Sterilisation mit Gas jedoch, dass nach der eigentlichen Sterilisation, bei der Entfernung des restlichen Ethylenoxids durch Einwirkung von Vakuum, ein Teil der Monomere in den Kartuschen verdampft, die dabei eine Gasphase in den Kartuschen ausbilden und somit einen Druck gegen die Kolben ausüben können. Das bedeutet, dass die Förderkolben unerwünscht in Richtung Kartuschenböden bewegt werden und im Extremfall aus den Kartuschen ausgestoßen werden, so dass die Pasten ausfließen können.

In der Medizin werden seit Jahrzehnten Polymethylmethacrylat-Knochenzemente zur dauerhaften mechanischen Fixierung von Totalgelenkendoprothesen eingesetzt. Diese basieren auf Pulver-Flüssigkeits-Systemen. Es wurden in jüngster Zeit auch Polymethylmethacrylat-Knochenzemente vorgeschlagen, die auf der Verwendung von Zementpasten beruhen (DE 10 2007 050 762 A1, DE 10 2008 030 312 A1, DE 10 2007 052 116 A1). Für derartige Zemente wurden bisher noch keine geeigneten Kartuschensysteme vorgeschlagen.

Bei der Anwendung von Knochenzementen zur Fixierung von Totalgelenkendoprothesen muss immer berücksichtigt werden, dass bei diesen Operationen das OP-Personal unter zeitlichem Druck steht. Bei medizinischen Anwendungen von Kartuschensystemen für die Applikation von pastenförmige Polymethylmethacrylat-Knochenzemente sollten diese daher grundsätzlich so gestaltet sein, dass sie weitgehend resistent gegenüber Anwenderfehlern sind und auch in Stresssituationen schnell und sicher bedient werden können.

Ein essentieller Bestandteil von pastenförmigen Polymethylmethacrylat-Knochenzementen ist das Monomer Methylmethacrylat. Dieses Monomer verdampft leicht und hat bei Raumtemperatur einen recht hohen Dampfdruck. Bei der Verwendung von Methylmethacrylat enthaltenden Pasten muss deshalb unbedingt beachtet werden, dass bei Vakuumeinwirkung, wie bei der Entgasung im Rahmen der Sterilisation mit Ethylenoxid, die Förderkolben in den Kartuschen durch das verdampfende Methylmethacrylat bewegt und im Extremfall aus den Kartuschen ausgestoßen werden können. Aus der DE 10 2005 041 962 B3 ist eine Kartusche bekannt, die zwei vorderseitige Verschlusskolben aufweist, die sich in eine Ausbringstellung verschieben lassen und dabei Auslassöffnungen öffnen. Die EP 1 728 560 A1 offenbart ein System zum Lagern und Applizieren einer Substanz, bei dem zwei Stopfen in einen geöffneten Bereich gedrückt werden, um ein Ausfließen der Komponenten zu ermöglichen.

Ein Kartuschensystem zum Mischen und Applizieren eines Mischguts ist aus der DE 10 2010 019 217 A1 bekannt. Das Kartuschensystem umfasst zumindest zwei Kartuschen und einen Mischraum, der mit den Kartuschen durch jeweils eine Öffnung verbunden ist. In den Kartuschen sind Förderkolben zum Austreiben der Ausgangskomponenten angeordnet. Ein verschiebbarer Verschluss ist in dem Mischraum angeordnet, der die Öffnungen verschließt. Durch Eindrehen eines Austragsrohrs wird der Verschluss im Mischraum verschoben und dadurch die Öffnungen freigelegt.

Nachteilig ist hieran, dass zunächst die Kartuschen geöffnet werden und erst später das Austragen der Kartuscheninhalte (Ausgangskomponenten) erfolgt. In der Zwischenzeit kann es zu einer Veränderung beziehungsweise Verschlechterung des Kartuscheninhalts kommen. Zudem kann von außen nicht gesehen werden, ob das Kartuschensystem schon geöffnet ist oder nicht.

Ein gattungsgemäßes Kartuschensystem ist aus der DE 10 2007 044 983 A1 bekannt. Das Kartuschensystem umfasst ein verschiebbares Ventil für jede Kartusche, das eine Öffnung in der Kartusche abdeckt. Durch Druckbeaufschlagung mit einem Förderkolben wird das Ventil verschoben und die Öffnung freigegeben. Das Ventil sitzt als Ring auf einem Zapfen am vorderen Ende des Kartuschensystems.

Nachteilig ist hieran, dass der Aufbau des Ventils komplex und daher kostenintensiv ist. Ein weiterer Nachteil ist auch hier, dass von außen nicht ohne weiteres erkennbar ist, ob der Verschluss schon geöffnet ist oder nicht. Grundsätzlich besteht immer ein Bedürfnis nach einer weiteren Vereinfachung des Aufbaus solcher Kartuschensysteme. Der Aufbau erfordert relativ kleine Querschnitte für die Durchführung der Ausgangskomponenten und des Mischguts, die zu einem hohen Widerstand beim Austragen des Kartuscheninhalts führen. Auch muss die Luft aus dem Ventilsystem verdrängt werden. Das Ventilsystem beinhaltet dabei einen Totraum, der weder zum Lagern noch zum Mischen der Komponenten gebraucht werden kann.

Aufgabe der Erfindung ist es, diese und weitere nicht genannte Nachteile zu überwinden. Insbesondere soll ein Kartuschensystem bereitgestellt werden, das einfach und kostengünstig in der Herstellung ist, gleichzeitig aber eine sichere und einfache Öffnung der Kartuschen gewährleistet. Eine sichere Aufbewahrung und ein sicherer Verschluss für pastenförmige Komponenten in zumindest zwei Kartuschen vor ihrer Verwendung sollen gewährleistet sein. Unmittelbar vor der Applikation der Pasten soll das Kartuschensystem mit möglichst geringem Aufwand sicher und schnell zu öffnen sein, um eine einfache Anwendung während Operationen zu ermöglichen und so die Probleme der bisherigen Kartuschensysteme und ihrer Verschlusssysteme zu vermindern beziehungsweise zu überwinden. Es soll also ein Verschlusssystem entwickelt werden, welches Kartuschen für mehrere Komponenten sicher verschließt und ein schnelles unkompliziertes Öffnen der einzelnen Kartuschen ermöglicht. Angestrebt wurde weiterhin ein Verschlusssystem das komplett aus kostengünstigen Kunststoffspritzgussteilen gefertigt werden kann.

Diese Aufgaben werden dadurch gelöst, dass zumindest zwei Kartuschen jeweils einen zylindrischen Hohlraum umfassen und die Verschlüsse im Inneren der Kartuschen angeordnet sind, wobei die Verschlüsse eine zylindrische Form haben und in ihrer Endposition in die zylindrischen Hohlräume der Kartuschen versenkbar sind, um die Öffnungen zu freizulegen.

Die Ausgangskomponenten sind erfindungsgemäß bevorzugt pastöse Massen, besonders bevorzugt Komponenten medizinischer Zemente.

Unter einer zylindrischen Form ist jeder Körper zu verstehen, der eine Grundfläche hat, die dem Querschnitt des Körpers in jeder Höhe vorzugsweise senkrecht zur Grundfläche entspricht. Eine zylindrische Form ist also von zwei parallelen, ebenen Flächen (Grund- und Deckfläche) und einer Mantel- beziehungsweise Zylinderfläche, die von parallelen Geraden gebildet wird, begrenzt. Das heißt, er entsteht durch Verschiebung einer ebenen Fläche oder Kurve entlang einer Geraden, die nicht in dieser Ebene liegt. Eine besonders bevorzugt Form des Zylinders ist die eines Zylinders mi kreisförmiger, ovaler oder abgerundeter Grundfläche.

Dabei kann vorgesehen sein, dass die zylindrischen Hohlräume der Kartuschen über zumindest eine Entlüftungsöffnung mit der Umgebung verbunden sind, so dass beim Versenken des Verschlusses die Luft aus dem Hohlraum entweicht, wobei vorzugsweise die Hohlräume im vorderen Bereich des Kartuschensystems angeordnet sind und die Entlüftungsöffnungen in Kartuschenköpfen der Kartuschen angeordnet sind.

Der vordere Bereich des Kartuschensystems ist der Bereich in dessen Richtung die Förderkolben bewegt werden, um die Ausgangskomponenten aus den Kartuschen auszutreiben. Die Kartuschenköpfe bilden das vordere Ende der Kartuschen.

Es ist erfindungsgemäß besonders bevorzugt, wenn die Hohlräume auf der Vorderseite durch die Kartuschenköpfe begrenzt sind, wobei insbesondere der Abstand der Öffnung zu den Kartuschenköpfen plus der Querschnitt der Öffnung in Längsrichtung der Kartusche größer ist als die Höhe der jeweiligen zylindrischen Verschlüsse in der jeweiligen Kartusche. Vorzugsweise entspricht die Höhe der zylindrischen Verschlüsse in den Kartuschen dem Abstand der Öffnung in der Kartusche zu dem Kartuschenkopf dieser Kartusche. Dann ist sichergestellt, dass die Verschlüsse in der versenkten Endposition die Öffnungen freigeben, vorzugsweise vollständig freigeben oder fast vollständig freigeben.

Nach einer weiteren Ausgestaltung der Erfindung kann vorgesehen sein, dass die Verschlüsse die Hohlräume von den mit den Ausgangskomponenten gefüllten Bereichen der Kartuschen trennen, vorzugsweise dicht trennen, besonders bevorzugt fluiddicht und/oder gasdicht trennen. Ganz besonders bevorzugt trennen die Verschlüsse die Hohlräume von den mit den Ausgangskomponenten gefüllten Bereichen der Kartuschen druckdicht.

Durch die Trennung wird erreicht, dass die Ausgangskomponenten bei Druckbeaufschlagung auf die Förderkolben sich nicht zwischen den Verschlüssen und den Kartuscheninnenwänden in den Hohlraum drücken lassen und bevorzugt auch keine Luft aus dem Hohlraum sich mit den Ausgangskomponenten mischt.

Des Weiteren kann vorgesehen sein, dass die verschiebbaren Verschlüsse in Presspassung in den Kartuschen angeordnet sind.

Auch diese Maßnahme sorgt für die Dichtigkeit der Kartuschen. Dabei muss bei der Presspassung darauf geachtet werden, dass sich die Förderkolben und die Verschlüsse noch manuell bewegen lassen. Bei geeigneter Presspassung kann auf zusätzliche Dichtungen verzichtet werden. Es kann auch vorgesehen sein, dass auch die Förderkolben in Presspassung in den Kartuschen angeordnet sind.

Ferner kann vorgesehen sein, dass die Verschlüsse kompakte Körper oder Hohlkörper sind, insbesondere Hohlzylinder mit zumindest einer verschlossenen Grundfläche sind.

Dieser Aufbau ist besonders einfach und kostengünstig zu realisieren. Zudem ist auch die Montage der Kartuschensysteme dann besonders einfach.

Gemäß einer weiteren besonders bevorzugten Ausgestaltung der Erfindung kann vorgesehen sein, dass die Hohlräume einen Innendurchmesser besitzen, der gleich oder größer dem Außendurchmesser des jeweiligen Verschlusses ist, und/oder die eine Länge von mindestens einem Zehntel der Höhe des Verschlusses haben.

Mit diesen Abmessungen kann der Verschluss gut in dem Hohlraum versenkt werden und es wird ein ausreichender Verschiebungsweg des Verschlusses sichergestellt, um Öffnungen mit ausreichender Querschnittsfläche im geöffneten Zustand zum Hindurchpressen der Ausgangskomponenten zu realisieren.

Erfindungsgemäße Kartuschensysteme können auch dadurch charakterisiert sein, dass in jeder Kartusche mindestens ein Sperrorgan angeordnet ist, das die axiale Bewegung des Verschlusses oberhalb der Auslassöffnung begrenzt.

Das Sperrorgan kann ein einfacher Anschlag sein oder eine andere Variation des Querschnitts des Kartuscheninnenraums oder ein oder mehrere Zapfen.

Ferner kann vorgesehen sein, dass in jeder Kartusche der Förderkolben über die in der Kartusche angeordnete Ausgangskomponente kraftschlüssig mit dem Verschluss dieser Kartusche verbunden ist, so dass durch Einwirken einer Kraft auf die Förderkolben die Kraft auf die Verschlüsse vermittelbar ist und die Verschlüsse von der Ausgangsposition in die Endposition überführbar sind.

Mit diesem Aufbau kann das Öffnen der Öffnungen beziehungsweise des Kartuschensystems und das Mischen und Applizieren des Mischguts durch Drücken auf die Förderkolben erfolgen. Es reicht also eine Betätigungsart beziehungsweise ein Arbeitsschritt, um das Kartuschensystem zu öffnen und um das Mischgut zu mischen und zu applizieren.

Bei einer weiteren besonders bevorzugten Ausgestaltung der Erfindung kann vorgesehen sein, dass die Förderkolben und die Verschlüsse jeweils paarweise in einem Rohr angeordnet sind, das dazwischen jeweils eine Ausgangskomponente enthält, wobei die verschlossenen Rohre in den Kartuschen angeordnet sind, insbesondere vom Kartuschenkopf aus gesehen hinter den Öffnungen.

Durch diesen Aufbau kann ein ungefülltes Bauteil für verschieden gefüllte Kartuschensysteme hergestellt werden. Die Kombination verschiedener Ausgangskomponenten erfolgt dann durch Einsetzen der Rohre, die mit unterschiedlichen Ausgangskomponenten befüllt sein können. Durch diese Maßnahme kann die Fertigung variabler gestaltet werden.

Bei einer weiteren Ausgestaltung der Erfindung kann vorgesehen sein, dass an zumindest einem Verschluss ein optischer Indikator angeordnet ist, durch den die Ausgangsposition von der Endposition durch mindestens eine Sichtöffnung und/oder ein Sichtfenster in zumindest einer Kartusche visuell erkennbar ist, insbesondere durch mindestens eine Sichtöffnung und/oder ein Sichtfenster in zumindest einem der Kartuschenköpfe, und/oder der durch diese mindestens eine Sichtöffnung aus der Kartusche herausragt, wenn der Verschluss sich in der Endposition befindet, wobei vorzugweise die Sichtöffnung auch die Entlüftungsöffnung ist.

Es kann erfindungsgemäß vorgesehen sein, dass jeweils am äußeren Ende eines als Verschlusskolben ausgebildeten Verschlusses ein optischer Indikator angebracht ist, der nach axialer Bewegung des Verschlusskolbens in Richtung der Kartuschenköpfe durch mindestens eine Öffnung in den Kartuschenköpfen visuell erkennbar ist oder durch diese mindestens eine Öffnung aus der Kartusche ausgetreten ist. Der optische Indikator ermöglicht es dem Anwender zu erkennen, wenn die Kartuschen durch Verschiebung der Verschlusskolben geöffnet sind.

Hierdurch kann der Anwender also unmittelbar erkennen, ob das Kartuschensystem schon verwendet wurde oder nicht. Der Einsatz eines bereits verwendeten Kartuschensystems ist im Operationsbetrieb leicht überprüfbar und kann leicht vermieden werden und dadurch kostbare Zeit sparen.

Auch kann vorgesehen sein, dass der Mischraum mit einem Austragsrohr mit statischem Mischer fest oder lösbar verbunden ist, wobei sich der statische Mischer vorzugsweise in den Mischraum erstreckt.

Das Austragsrohr erleichtert die Applikation des Mischguts und der statische Mischer verbessert die Durchmischung des Mischguts.

Eine weitere besonders bevorzugte Ausgestaltung eines erfindungsgemäßen Kartuschensystems kann dadurch realisiert werden, dass vorgesehen ist, dass eine drehbare Arretierungsvorrichtung drehbar in einer Halterung im Bereich der Kartuschenköpfe gelagert ist, wobei die Arretierungsvorrichtung ein rotationssymmetrischer Körper mit zumindest einer zylindrischen Ausnehmung ist, vorzugsweise mit einer Ausnehmung für jede Kartusche, wobei die Arretierungsvorrichtung zumindest bereichsweise zumindest einen Kartuschenkopf bildet und die Ausnehmung der Arretierungsvorrichtung in einer ersten Position zumindest einen Teil eines der Hohlräume zur Aufnahme des Verschlusses bildet, so dass der Verschluss dieser Kartusche in dieser ersten Position in den Hohlraum versenkbar ist und in einer zweiten, gedrehten Position der Arretierungsvorrichtung nicht in den Hohlraum versenkbar ist.

Die zylindrischen Ausnehmungen sind vorzugsweise senkrecht zur Symmetrieachse des rotationssymmetrischen Körpers angeordnet. Nur der Grundkörper der Arretierungsvorrichtung ist ein rotationssymmetrischer Körper. Die Symmetrie der Arretierungsvorrichtung ist durch die Ausnehmungen und eventuell weitere Durchführungen und/oder Öffnungen, zum Durchpressen der Ausgangskomponenten oder des Mischguts, und/oder als Entlüftungsöffnungen und/oder Sichtfenster gebrochen.

Die erste und die zweite Position werden durch Drehen der Arretierungsvorrichtung ineinander überführt. Die Drehachse ist dabei die Symmetrieachse des rotationssymmetrischen Körpers der Arretierungsvorrichtung. Vorzugsweise ist die Drehachse senkrecht zur Längsachse der Kartuschen angeordnet.

Auch wenn diese Ausführungsform eine Entarretierung des Kartuschensystems und damit einen zusätzlichen Arbeitsschritt erforderlich macht, bietet sie den Vorteil, dass ein versehentliches Öffnen des Kartuschensystems durch einen ungewollten Druck auf die Förderkolben vermieden werden kann. Die arretierte Position der Arretierungsvorrichtung ist die zweite Position und die einsatzbereite Position der Arretierungsvorrichtung ist die erste Position. In der einsatzbereiten ersten Position können die Verschlüsse in den Hohlräumen versenkt werden, in der arretierten zweiten Position nicht.

Ferner kann auch vorgesehen sein, dass ein Austragsrohr an der Arretierungsvorrichtung angeordnet ist und über eine Durchführung durch die Arretierungsvorrichtung mit dem Mischraum in der ersten Position verbunden ist, wobei vorzugsweise die Auslassöffnung des Mischraums durch die Arretierungsvorrichtung in der zweiten Position verschlossen ist.

Ein erfindungsgemäßes Mehrkomponentenkartuschensystem mit parallel zueinander angeordneten Kartuschen, die jeweils einen pastösen Stoff enthalten und mit einem Austragsrohr mit statischem Mischer irreversibel oder reversibel verbunden sind, kann also beispielsweise dadurch realisiert sein, dass
a) jede Kartusche (am Kartuschenboden) mit mindestens einem in axialer Richtung verschiebbaren Förderkolben verschlossen ist,
b) jede Kartusche am Kartuschenkopf mindestens eine Öffnung besitzt, die senkrecht zur Längsachse der Kartusche angeordnet ist,
c) die Öffnung oder Öffnungen jeder Kartusche mit mindestens einem axial verschiebbaren Verschlusskolben verschlossen ist, wobei die Öffnung oder Öffnungen durch eine Verschiebung des Verschlusskolbens oder der Verschlusskolben freigegeben werden können,
d) die Kartuschen so miteinander verbunden sind, dass diese einen Mischraum bilden, der in Richtung der Kartuschenböden verschlossen ist,
e) die Öffnung oder Öffnungen den Innenraum der Kartuschen mit dem Mischraum verbinden,
f) der Mischraum mit dem Austragsrohr irreversibel oder reversibel durchgängig verbunden ist,
g) an jedem Kartuschenkopf oberhalb der Öffnung oder Öffnungen ein Hohlraum vorhanden ist, der einen Innendurchmesser besitzt, der gleich oder größer dem Außendurchmesser des Verschlusskolbens ist, und der eine Länge von mindestens einem Zehntel der Höhe des Verschlusskolbens hat,
h) an jedem Kartuschenkopf mindestens ein Sperrorgan angeordnet ist, das die axiale Bewegung des Verschlusskolbens oberhalb der Öffnung oder Öffnungen begrenzt, und
i) in jeder Kartusche der Förderkolben über den in der Kartusche angeordneten pastösen Stoff kraftschlüssig mit dem Verschlusskolben verbunden ist.

Die Verschlüsse können erfindungsgemäß als Verschlusskolben ausgeführt sein.

Eine weitere Ausgestaltungsvariante der Erfindung kann vorsehen, dass jeweils ein Förderkolben und ein Verschlusskolben in einem Rohr angeordnet sind, das einen pastösen Stoff als Ausgangskomponente enthält, wobei dieses verschlossene Rohr in der Kartusche unterhalb der Auslassöffnung angeordnet ist. Das bedeutet, dass das Rohr mit den Förderkolben und den Verschlusskolben das Primärpackmittel des pastösen Stoffs bildet. Dieses Primärpackmittel wird in die Kartuschen eingesetzt. Dadurch ist eine separate Abfüllung von unterschiedlichsten Ausgangskomponenten möglich, die in beliebiger Weise in der Mehrkomponentenkartusche miteinander kombiniert werden können. Auf diese Weise können unterschiedlichste Kombinationen von pastösen Knochenzementen mit oder ohne darin enthaltene Antibiotika abgepackt werden.

Der Erfindung liegt die überraschende Erkenntnis zugrunde, dass einfache Zylinder als verschiebbare Verschlüsse in den Kartuschen zum Verschließen von Öffnungen zum Durchpressen von Ausgangskomponenten eines Mischguts verwendet werden können, wobei die Verschlüsse in Hohlräumen der Kartuschen versenkbar sind, um das Kartuschensystem zu öffnen und einsatzbereit zu machen. Die Vorzüge der Erfindung liegen dabei unter anderem in der Einfachheit ihrer Ausführung. Gleichzeitig ist es mit der vorliegenden Erfindung möglich, ein Kartuschensystem zu realisieren, bei dem die Öffnung des Kartuschensystems mit dem gleichen Druck auf die Förderkolben erfolgt, der zum Mischen und Austreiben des Mischguts beziehungsweise der Ausgangskomponenten aus den Kartuschen in den Mischraum genutzt wird.

Durch die Erfindung gelingt es, ein Mehrkomponentenkartuschensystem zur Verfügung zu stellen, bei dem die einzelnen Kartuschen automatisch und synchron bei Druckbeaufschlagung der Förderkolben geöffnet werden.

Im Folgenden werden Ausführungsbeispiele der Erfindung anhand von sechs schematisch dargestellten Figuren erläutert, ohne jedoch dabei die Erfindung zu beschränken. Dabei zeigt:
- Figur 1:: eine schematische Querschnittansicht eines erfindungsgemäßen Kartuschensystems mit geschlossenen Öffnungen zum Mischraum;
- Figur 2:: eine schematische Querschnittansicht eines erfindungsgemäßen Kartuschensystems mit geöffneten Öffnungen zum Mischraum;
- Figur 3:: eine schematische Querschnittansicht eines erfindungsgemäßen geschlossenen Kartuschensystems mit Indikator;
- Figur 4:: eine schematische Querschnittansicht eines erfindungsgemäßen geöffneten Kartuschensystems mit Indikator;
- Figur 5:: eine schematische Querschnittansicht eines erfindungsgemäßen Kartuschensystems mit eingebrachten Rohren; und
- Figur 6:: eine schematische Querschnittansicht eines erfindungsgemäßen geschlossenen Kartuschensystems mit drehbarer Arretierungsvorrichtung.

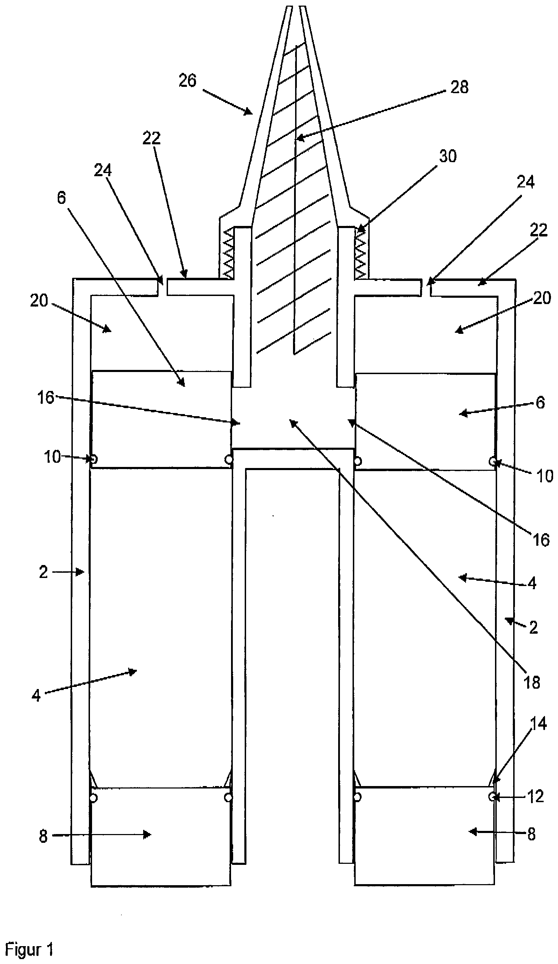

Figur 1 zeigt eine schematische Querschnittansicht eines erfindungsgemäßen Kartuschensystems mit zwei Kartuschen 2. Die Kartuschen 2 bilden zylindrische Innenräume. Im Inneren der Kartuschen 2 sind Ausgangskomponenten 4 für ein zu mischendes Mischgut enthalten. Die Ausgangskomponenten 4 können beispielsweise pastöse Massen sein und sind bevorzugt zur Bildung eines medizinischen Zements als Mischgut vorgesehen. Die Bereiche der Kartuschen 2, in denen die Ausgangskomponenten 4 enthalten sind, sind auf der Vorderseite (in allen Figuren oben) durch jeweils einen Verschluss 6 begrenzt und auf der Rückseite durch jeweils einen Förderkolben 8 abgeschlossen. Die Verschlüsse 6 haben die zylindrische Geometrie des Inneren der Kartuschen 2 und sind mit Dichtungen 10, die die Verschlüsse 6 vollumfänglich umgeben, abgedichtet in den Innenräumen der Kartuschen 6 angeordnet. Auch die Förderkolben 8 haben die zylindrische Geometrie der Innenräume der Kartuschen 2 und dichten diese mit Dichtungen 12 ab. Die Dichtungen 12 umgeben die Förderkolben 8 ebenfalls vollumfänglich.

Sowohl die Förderkolben 8 als auch die Verschlüsse 6 sind beweglich entlang der Längsachse der Kartuschen 2 angeordnet (in allen Figuren von unten nach oben beziehungsweise umgekehrt). Die Ausgangskomponenten 4 sind inkompressible Massen. Dies führt dazu, dass bei Beaufschlagung einer Kraft auf die Förderkolben 8, die diese tiefer ins Innere der Kartuschen 2 drückt, die Kraft über die Ausgangskomponenten 4 hydraulisch an die Verschlüsse 6 weitergeleitet wird. Damit die Förderkolben 8 nur synchron bewegt werden können, können diese miteinander fest verbunden sein. An den Förderkolben 8 sind Abstreiflippen 14 angeordnet, die dazu dienen, dass die Ausgangskomponenten 4 durch die Förderkolben 8 möglichst vollständig nach vorne pressbar sind.

Die Verschlüsse 6 verschließen Öffnungen 16, die das Innere der Kartuschen 2 mit einem Mischraum 18 verbinden. Auf der Vorderseite der Kartuschen 2 wird der Innenraum der Kartuschen 2 durch jeweils einen Hohlraum 20 gebildet, der auf der Vorderseite durch einen Kartuschenkopf 22 abgeschlossen ist. Gegenüber dem Kartuschenkopf 22 ist der Kartuschenboden auf der Rückseite des Kartuschensystems, der im Ausgangszustand durch die Förderkolben 8 gebildet wird. Im Kartuschenkopf 22 befindet sich eine Entlüftungsöffnung 24, durch die der Hohlraum 20 mit der Umgebung verbunden ist.

Der Mischraum 18 ist an der Vorderseite über eine Auslassöffnung geöffnet, wobei auf dem Mischraum 18 beziehungsweise auf der Auslassöffnung des Mischraums 18 ein Austragsrohr 26 angeordnet ist. Im Austragsrohr 26 ist ein statischer Mischer 28 angeordnet, der bis in den Mischraum 18 reicht. Wenn die Ausgangskomponenten 4 aus den Kartuschen 2 durch die Öffnungen 16 in den statischen Mischer 28 gepresst werden, so werden diese durch den statischen Mischer 28 durchmischt, um ein gut vermischtes Mischgut zu erhalten, das anschließend appliziert werden kann. Das Austragsrohr 26 ist über ein Gewinde 30 auf der Vorderseite des Kartuschensystems aufgeschraubt.

Figur 1 zeigt die Verschlüsse 6 in der Ausgangsposition, in der die Öffnungen 16 durch die Verschlüsse 6 verschlossen sind. Wird ein Druck auf die Förderkolben 8 ausgeübt, so wird der Druck über die zumindest weitgehend inkompressiblen Ausgangskomponenten 4 auf die Verschlüsse 6 weitergeleitet. Dadurch werden die Verschlüsse 6 in die Hohlräume 20 gedrückt. Die Luft aus den Hohlräumen 20 kann durch die Entlüftungsöffnungen 24 entweichen. Durch die Bewegung der Verschlüsse 6 werden die Öffnungen 16 freigelegt.

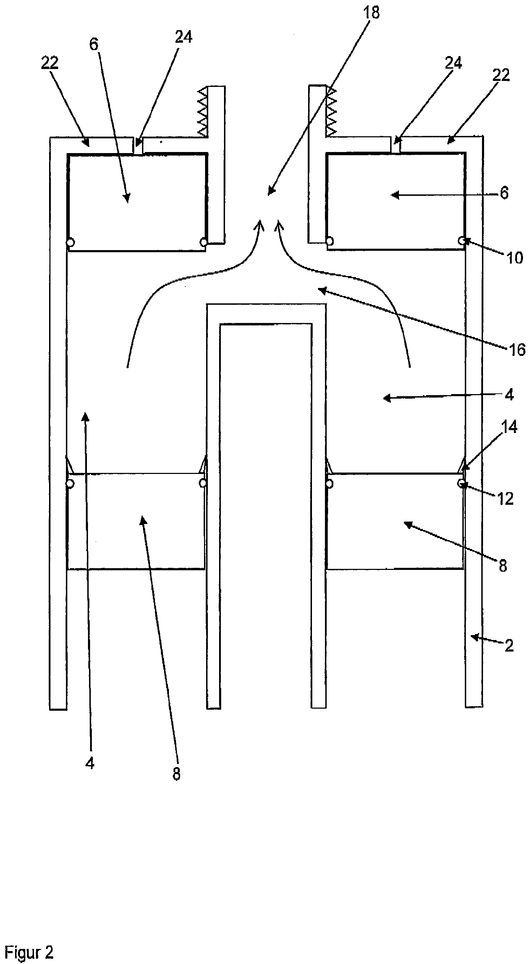

Die geöffnete Situation ist in Figur 2 dargestellt, in der eine schematische Querschnittansicht des erfindungsgemäßen Kartuschensystems mit geöffneten Öffnungen 16 zum Mischraum 18 gezeigt ist. Die Verschlüsse 6 befinden sich dort in der Endposition beziehungsweise der geöffneten Position. Der Druck auf die Förderkolben 8 bewirkt nunmehr, dass die Ausgangskomponenten 4 für das Mischgut durch die Öffnungen 16 in den Mischraum 18 gedrückt werden und anschließend aus dem Mischraum 18 durch die Auslassöffnung hinaus.

In den Figuren 2, 3 und 4 ist der Aufbau der Einfachheit halber ohne das Austragsrohr 26 mit dem statischen Mischer 28 gezeigt. Der Mischraum 18 ist vorne im Bereich seiner Auslassöffnung offen. Das Austragsrohr 26 muss auch nicht aufgeschraubt werden, sondern kann über eine Arretierungsvorrichtung mit dem Mischraum 18 verbunden sein oder einteilig fest mit dem Mischraum 18 ausgeführt sein.

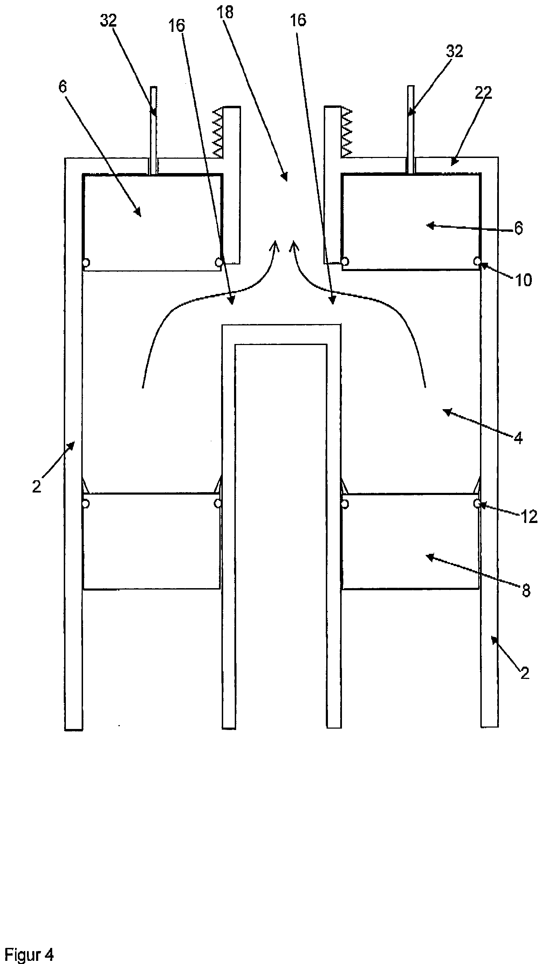

Die Ausgangskomponenten 4 vermischen sich im Mischraum 18 und werden durch das Austragsrohr 26 gepresst und dort durch den statischen Mischer 28 gut durchmischt. Der Fluss der Ausgangskomponenten 4 aus den Kartuschen 2 durch die Öffnungen 16 und durch den Mischraum 18 ist in Figur 2 und in Figur 4 durch die gebogenen Pfeile dargestellt. Die selbe Kraft, die zum Öffnen der Öffnungen 16 durch Versenken der Verschlüsse 6 in den Hohlräumen 20 dient, wird also auch zum Austragen und Mischen der Ausgangskomponenten 4 verwendet, so dass das Kartuschensystem in einem Arbeitsschritt einsatzbereit gemacht und angewendet werden kann.

Gleichzeitig ist der Aufbau denkbar einfach. Alle Teile können durch einfache Spritzgießtechniken aus Kunststoff hergestellt werden und sind leicht zusammensetzbar. Der Verschluss 6 kann ein einfacher Zylinder sein, der auch Hohl ausgeführt sein kann. Das gezeigte Kartuschensystem, leistet also einfachste Bedienbarkeit bei gleichzeitig einfachstem und damit kostengünstigem Aufbau.

Die Oberseite der Verschlüsse 6 oder die gesamten Verschlüsse 6 können in einer Farbe ausgeführt sein, die einen starken Kontrast zur äußeren Farbe des Kartuschensystems hat. Dadurch kann erreicht werden, dass in der Endposition der Verschlüsse 6 diese gut durch die Entlüftungsöffnungen 24 erkennbar sind. Der Kontrast dient dann als Indikator für den Zustand des Kartuschensystems. So kann auf einfach Weise von außen erkannt werden, ob das Kartuschensystem schon benutzt wurde oder nicht.

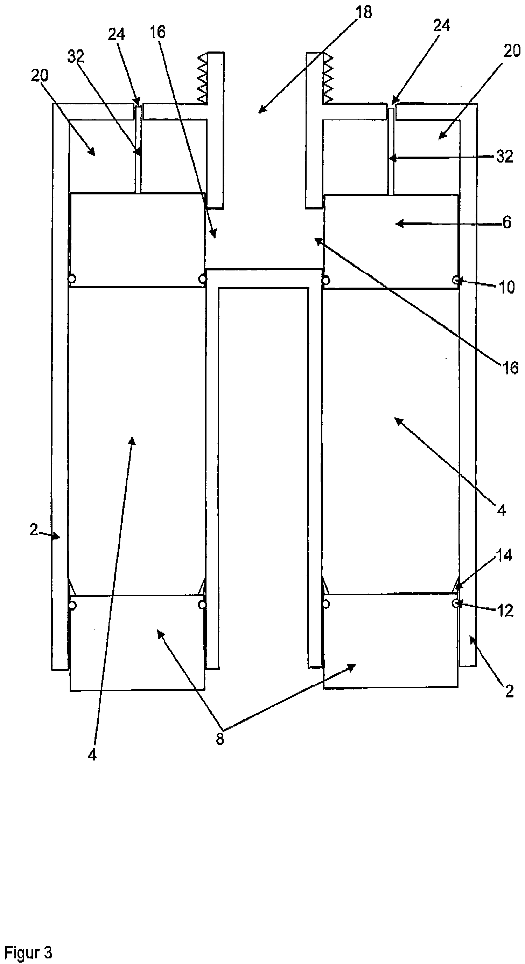

Ein noch leichter erkennbarer Indikator 32 ist in einer Ausführungsform nach den Figuren 3 und 4 dargestellt, die eine schematische Querschnittansicht eines erfindungsgemäßen Kartuschensystems in offenem und geschlossenen Zustand zeigen. Der Indikator 32 ist als Stäbchen ausgebildet, der auf der Vorderseite der Verschlüsse 6 angeordnet ist und in die (Figur 3) oder durch die (Figur 4) Entlüftungsöffnungen 24 reicht. Vorzugsweise hat der Indikator 32 wieder eine Signalfarbe, wie beispielsweise Rot oder Orange, mit einem guten Kontrast zum restlichen Äußeren des Kartuschensystems, insbesondere der Kartuschenköpfe 22 außen.

Der Indikator 32 sollte entweder einen kleineren Durchmesser als die Entlüftungsöffnungen 24 aufweisen, damit Luft aus dem Hohlraum 20 durch die verbleibenden Spalten der Entlüftungsöffnungen 24 entweichen kann, oder es sind weitere Entlüftungsöffnungen (nicht gezeigt) vorgesehen, durch die Luft aus dem Hohlraum 20 austreten kann. Die Entlüftung des Hohlraums 20 bewirkt, dass über die Förderkolben 8 keine Kraft gegen den sich aufbauenden Druck im Hohlraum 20 aufgebracht werden muss.

Es kann jedoch erfindungsgemäß auch vorgesehen sein, dass keine Entlüftungsöffnungen vorgesehen sind, so dass die komprimierte Luft in den Hohlräumen 20 eine Rückstellkraft bewirkt, die die Verschlüsse 6 wieder in die Ausgangsposition zurücktreibt, beziehungsweise zum Schließen der Öffnungen 16 führt, wenn keine Kraft mehr von außen auf die Förderkolben 8 ausgeübt wird. Hierzu können auch Federelemente (nicht gezeigt) in den Hohlräumen 20 angeordnet sein. Bevorzugt wird jedoch die Einmalanwendung mit Entlüftungsöffnungen 24.

Im geöffneten Zustand der Verschlüsse 6 ragen die Indikatoren 32 aus den Entlüftungsöffnungen 24 heraus und sind gut sichtbar.

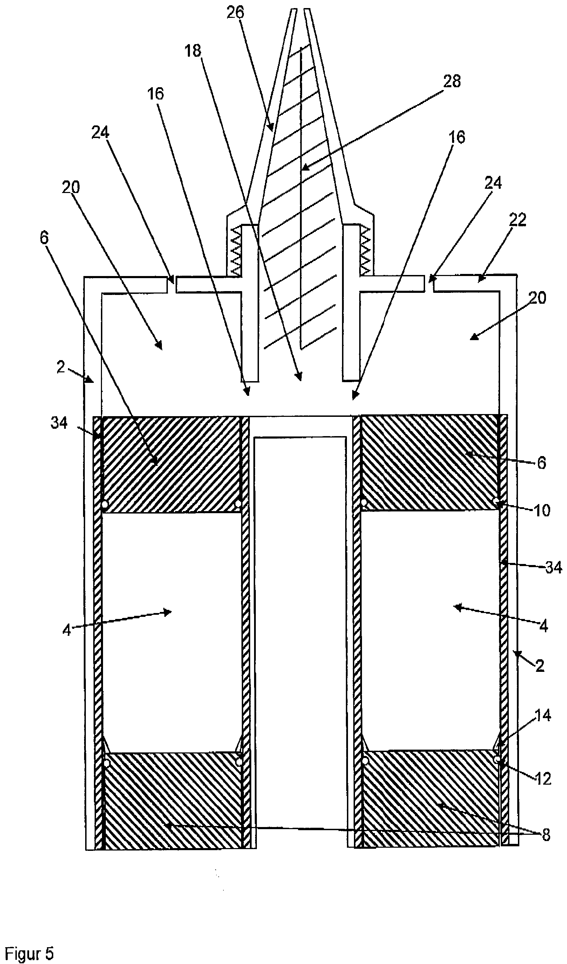

Figur 5 zeigt ein weiteres Ausführungsbeispiel eines alternativen erfindungsgemäßen Kartuschensystems in schematischer Querschnittansicht, bei dem Rohre 34 in die Kartuschen 2 eingesteckt sind. Die Rohre 34 enthalten sowohl die Förderkolben 8, als auch die Verschlüsse 6 und die Ausgangskomponenten 4. Zur Fertigung des Kartuschensystems können verschiedene Rohre 34 mit unterschiedlichen Ausgangskomponenten 4 kombiniert und in ein vorgefertigtes Standardteil eingesetzt werden.

Die Einsätze umfassend die Rohre 34 sind in Figur 5 schraffiert gekennzeichnet. Der Vorteil dieses Systems ist also eine weitere Vereinfachung der Fertigung erfindungsgemäßer Kartuschensysteme. Ansonsten bleibt das Funktionsprinzip das gleiche. Die Verschlüsse 6 werden durch Ausüben einer Kraft auf die Förderkolben 8 nach vorne an den Öffnungen 16 vorbei in den Hohlraum 20 gedrückt. Dadurch wird eine Verbindung zwischen dem Inneren der Rohre 34 mit dem Kartuscheninhalt 4 (also den Ausgangskomponenten 4) und dem Mischraum 18 über die Öffnungen 16 erzeugt. Durch die Öffnungen 16 werden die Ausgangskomponenten 4 in den Mischraum 18 gepresst und dort vermischt. Anschließend erfolgt durch den statischen Mischer 28 eine zusätzliche Durchmischung der Ausgangskomponenten 4 und das Mischgut kann anschließend über das Austragsrohr 26 aufgetragen werden. Beispielsweise könnte so ein fertiger Knochenzement während einer Operation appliziert werden.

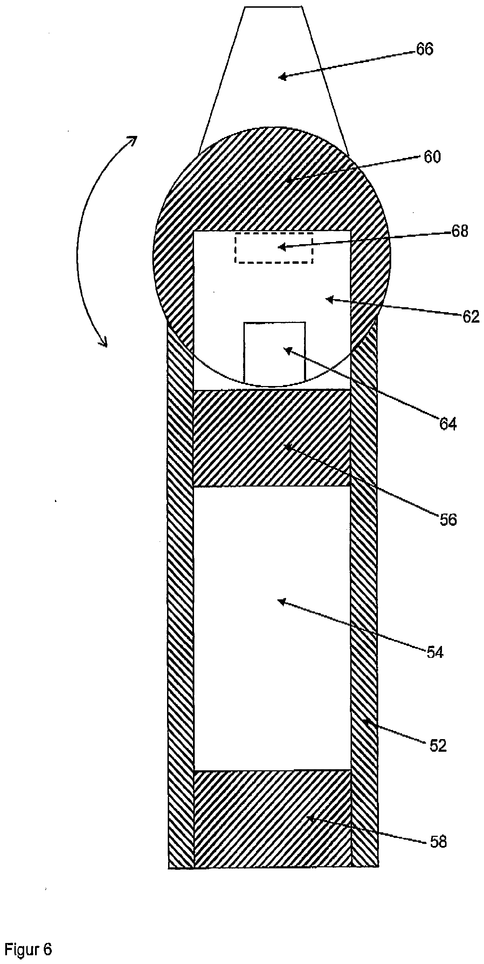

Figur 6 zeigt eine schematische Querschnittansicht eines weiteren erfindungsgemäßen Kartuschensystems in geschlossenem Zustand. Der gezeigte Querschnitt ist parallel zu einer anderen Seite des Kartuschensystems als die in den Figuren 1 bis 5 gezeigten Querschnitte. Bezogen auf die Figuren 1 bis 5 würde der Querschnitt hier senkrecht zu den gezeigten Querschnitten von oben nach unten verlaufen.

Der Querschnitt läuft durch eine erste Kartusche 52, die zweite Kartusche ist hinter dieser Kartusche 52 angeordnet und daher nicht zu sehen. Alle geschnittenen Körper sind in Figur 6 schraffiert gekennzeichnet. Im Inneren der Kartusche 52 ist eine flüssige oder pastöse Ausgangskomponente 54 für ein Mischgut enthalten. Der zylindrische Innenraum der Kartusche 52, in dem die Ausgangskomponente 54 enthalten ist, ist auf der Vorderseite (in Figur 6 oben) durch einen Verschluss 56 verschlossen und auf der Rückseite durch einen Förderkolben 58. Der Verschluss 56 und der Förderkolben 58 sitzen in Presspassung in der Kartusche 52 und schließen diese ab. Durch die Presspassung ist das Innere der Kartusche 52 mit der Ausgangskomponente 54 hinreichend angedichtet, so dass auf Dichtungen am Verschluss 56 und dem Förderkolben 58 verzichtet werden kann.

Als Kartuschenkopf ist eine drehbare Arretierungsvorrichtung 60 in einer Halterung (nicht gezeigt) am vorderen Ende des Kartuschensystems angeordnet, wobei die drehbare Arretierungsvorrichtung 60 eine Ausnehmung 62 umfasst. Die Arretierungsvorrichtung 60 ist in Figur 6 im offenen Zustand gezeigt, in der die Ausnehmung 62 den wesentlichen Teil eines Hohlraums 62 zum Versenken des Verschlusses 56 bildet. Wenn die Arretierungsvorrichtung 60 verdreht ist, verhindern die Wände der Arretierungsvorrichtung 60, dass der Verschluss 56 in den Hohlraum 62 versenkt werden kann, so dass das Kartuschensystem nur in dem gezeigten Zustand angewendet werden kann.

In der Arretierungsvorrichtung 60 und der Kartuschenwand, auf die in Figur 6 geblickt wird, ist eine Öffnung 64 vorgesehen, die durch den Verschluss 56 verschlossen oder geöffnet werden kann. Wenn eine Kraft auf den Förderkolben 58 wirkt, wird diese Kraft über die Ausgangskomponente 54 auf den Verschluss 56 übertragen. In der gezeigten Position der Arretierungsvorrichtung 60 wird der Verschluss 56 in den Hohlraum 62, vorbei an der Öffnung 64 geschoben. Der Kartuscheninhalt 54 kann schließlich durch die Öffnung 64 aus der Kartusche 52 in einen dahinter gelegenen Mischraum (nicht gezeigt) fließen, wo sie mit einer anderen Ausgangskomponente aus der zweiten Kartusche gemischt wird.

Die Arretierungsvorrichtung 60 ist fest mit einem Austragsrohr 66 verbunden, so dass sich das Austragsrohr 66 mit der Arretierungsvorrichtung 60 dreht. Der Mischraum wird zumindest bereichsweise durch die Arretierungsvorrichtung 60 begrenzt oder ist komplett darin ausgebildet.

Die Arretierungsvorrichtung 60 ist als rechtwinkliger Zylinder mit kreisförmiger Grundfläche ausgebildet, in dem die Ausnehmungen 62, der Mischraum und die Öffnungen 64 als Verbindungen zu dem Mischraum für jede der Kartuschen 52 enthalten sind. Des Weiteren kann eine Durchführung zum Austragsrohr 66 und Entlüftungsöffnungen (nicht gezeigt) in der Arretierungsvorrichtung 60 vorgesehen sein. Die Arretierungsvorrichtung 60 kann um ihre Zylinderachse gedreht werden, wie in Figur 6 durch die Pfeile auf der linken Seite angedeutet, um die Arretierungsvorrichtung 60 von der geschlossenen Position in die geöffnete Position (in Figur 6 gezeigt) zu überführen. Die Arretierungsvorrichtung 60 kann nach Art einer Walze ausgebildet sein.

Die Arretierungsvorrichtung 60 bietet eine zusätzliche Sicherheit, dass die Förderkolben 58 nicht unbedacht eingedrückt werden können und das Kartuschensystem so geöffnet wird. Dafür muss ein zusätzlicher Arbeitsschritt vollzogen werden, um das Kartuschensystem einsatzbereit zu machen.

Ein Sichtfenster 68 ist auf der Seite vorgesehen, durch das der Verschluss 56 in der versenkten Endposition im Hohlraum 62 zu erkennen ist. Das Sichtfenster 68 liegt eigentlich oberhalb des gezeigten Querschnitts und ist deswegen mit einer gestrichelten Linie gekennzeichnet. Das Sichtfenster 68 kann mit einem durchsichtigen Kunststoff abgedeckt sein, so dass es ausschließlich zur Indikation des Zustands des Kartuschensystems verwendet werden kann, oder das Sichtfenster 68 ist offen und dient gleichzeitig als Entlüftungsöffnung 68.

Die zylindrische Arretierungsvorrichtung 60 bildet also den vorderen Teil der Kartuschen 52 und ist als beweglicher Teil derselben aufzufassen.

Die in der voranstehenden Beschreibung, sowie den Ansprüchen, Figuren und Ausführungsbeispielen offenbarten Merkmale der Erfindung können sowohl einzeln, als auch in jeder beliebigen Kombination für die Verwirklichung der Erfindung in ihren verschiedenen Ausführungsformen wesentlich sein.

### Bezugszeichenliste

- 2, 52: Kartusche
- 4, 54: Ausgangskomponente für Mischgut
- 6, 56: Verschluss
- 8, 58: Förderkolben
- 10, 12: Dichtung
- 14: Abstreiflippe
- 16, 64: Öffnung
- 18: Mischraum
- 20, 62: Hohlraum / Ausnehmung
- 22: Kartuschenkopf
- 24: Entlüftungsöffnung
- 26, 66: Austragsrohr
- 28: statischer Mischer
- 30: Gewinde
- 32: Indikator
- 34: Rohr
- 60: Arretierungsvorrichtung
- 68: Entlüftungsöffnung / Sichtfenster

## Patentansprüche

1. Kartuschensystem zum Mischen und Applizieren eines Mischguts, insbesondere eines medizinischen Zements, umfassend zumindest zwei parallel zueinander angeordnete Kartuschen (2, 52), **gekennzeichnet durch**
einen Mischraum (18) mit einer Auslassöffnung, wobei die Kartuschen (2, 52) jeweils zumindest eine Öffnung (16, 64) in den Kartuschenwänden umfassen, die die Kartuschen (2, 52) mit dem Mischraum (18) verbinden, die Kartuschen (2, 52) jeweils einen Förderkolben (8, 58) zum Austreiben von Ausgangskomponenten (4, 54) des Mischguts aus den Kartuschen (2, 52) **durch** die Öffnungen (16, 64) umfassen und jeder Kartusche (2, 52) zumindest ein in Längsrichtung der Kartuschen (2, 52) verschiebbarer Verschluss (6, 56) zugeordnet ist, wobei die Verschlüsse (6, 56) die Öffnungen (16, 64) der Kartuschen (2, 52) in einer Ausgangsposition verschließen und die Öffnungen (16, 64) in einer Endposition der verschiebbaren Verschlüsse (6, 56) zumindest bereichsweise geöffnet sind, undzumindest zwei Kartuschen (2, 52) jeweils einen zylindrischen Hohlraum (20, 62) umfassen und die Verschlüsse (6, 56) im Inneren der Kartuschen (2, 52) angeordnet sind, wobei die Verschlüsse (6, 56) eine zylindrische Form haben und in ihrer Endposition in die zylindrischen Hohlräume (20, 62) der Kartuschen (2, 52) versenkbar sind, um die Öffnungen (16, 64) zu freizulegen.

2. Kartuschensystem nach Anspruch 1, **dadurch gekennzeichnet, dass** die zylindrischen Hohlräume (20, 62) der Kartuschen (2, 52) über zumindest eine Entlüftungsöffnung (24, 68) mit der Umgebung verbunden sind, so dass beim Versenken des Verschlusses (6, 56) die Luft aus dem Hohlraum (20, 62) entweicht, wobei vorzugsweise die Hohlräume (20, 62) im vorderen Bereich des Kartuschensystems angeordnet sind und die Entlüftungsöffnungen (24, 68) in Kartuschenköpfen (22) der Kartuschen (2, 52) angeordnet sind.

3. Kartuschensystem nach Anspruch 1 oder 2, **dadurch gekennzeichnet, dass** die Verschlüsse (6, 56) die Hohlräume (20, 62) von den mit den Ausgangskomponenten (4, 54) gefüllten Bereichen der Kartuschen (2, 52) trennen, vorzugsweise dicht trennen, besonders bevorzugt fluiddicht und/oder gasdicht trennen.

4. Kartuschensystem nach einem der vorangehenden Ansprüche, **dadurch gekennzeichnet, dass**
die verschiebbaren Verschlüsse (6, 56) in Presspassung in den Kartuschen (2, 52) angeordnet sind.

5. Kartuschensystem nach einem der vorangehenden Ansprüche, **dadurch gekennzeichnet, dass**
die Verschlüsse (6, 56) kompakte Körper oder Hohlkörper sind, insbesondere Hohlzylinder mit zumindest einer verschlossenen Grundfläche sind.

6. Kartuschensystem nach einem der vorangehenden Ansprüche, **dadurch gekennzeichnet, dass**
die Hohlräume (20, 62) einen Innendurchmesser besitzen, der gleich oder größer dem Außendurchmesser des jeweiligen Verschlusses (6, 56) ist, und/oder die eine Länge von mindestens einem Zehntel der Höhe des Verschlusses (6, 56) haben.

7. Kartuschensystem nach einem der vorangehenden Ansprüche, **dadurch gekennzeichnet, dass**
in jeder Kartusche (2, 52) mindestens ein Sperrorgan angeordnet ist, das die axiale Bewegung des Verschlusses (6, 56) oberhalb der Auslassöffnung begrenzt.

8. Kartuschensystem nach einem der vorangehenden Ansprüche, **dadurch gekennzeichnet, dass**
in jeder Kartusche (2, 52) der Förderkolben (8, 58) über die in der Kartusche (2, 52) angeordnete Ausgangskomponente (4, 54) kraftschlüssig mit dem Verschluss (6, 56) dieser Kartusche (2, 52) verbunden ist, so dass durch Einwirken einer Kraft auf die Förderkolben (8, 58) die Kraft auf die Verschlüsse (6, 56) vermittelbar ist und die Verschlüsse (6, 56) von der Ausgangsposition in die Endposition überführbar sind.

9. Kartuschensystem nach einem der vorangehenden Ansprüche, **dadurch gekennzeichnet, dass**
die Förderkolben (8, 58) und die Verschlüsse (6, 56) jeweils paarweise in einem Rohr (34) angeordnet sind, das dazwischen jeweils eine Ausgangskomponente (4, 54) enthält, wobei die verschlossenen Rohre (34) in den Kartuschen (2, 52) angeordnet sind, insbesondere vom Kartuschenkopf (22) aus gesehen hinter den Öffnungen (16, 64).

10. Kartuschensystem nach einem der vorangehenden Ansprüche, **dadurch gekennzeichnet, dass**
an zumindest einem Verschluss (6, 56) ein optischer Indikator (32) angeordnet ist, durch den die Ausgangsposition von der Endposition durch mindestens eine Sichtöffnung (24, 68) und/oder ein Sichtfenster (68) in zumindest einer Kartusche (2, 52) visuell erkennbar ist, insbesondere durch mindestens eine Sichtöffnung (24, 68) und/oder ein Sichtfenster (68) in zumindest einem der Kartuschenköpfe (22), und/oder der durch diese mindestens eine Sichtöffnung (24, 68) aus der Kartusche (2, 52) herausragt, wenn der Verschluss (6, 56) sich in der Endposition befindet, wobei vorzugweise die Sichtöffnung (24, 68) auch die Entlüftungsöffnung (24, 68) ist.

11. Kartuschensystem nach einem der vorangehenden Ansprüche, **dadurch gekennzeichnet, dass**
der Mischraum (18) mit einem Austragsrohr (26, 66) mit statischem Mischer (28) fest oder lösbar verbunden ist, wobei sich der statische Mischer (28) vorzugsweise in den Mischraum (18) erstreckt.

12. Kartuschensystem nach einem der vorangehenden Ansprüche, **dadurch gekennzeichnet, dass**
eine drehbare Arretierungsvorrichtung (60) drehbar in einer Halterung im Bereich der Kartuschenköpfe gelagert ist, wobei die Arretierungsvorrichtung (60) ein rotationssymmetrischer Körper mit zumindest einer zylindrischen Ausnehmung (62) ist, vorzugsweise mit einer Ausnehmung für jede Kartusche (52), wobei die Arretierungsvorrichtung (60) zumindest bereichsweise zumindest einen Kartuschenkopf (22) bildet und die Ausnehmung (62) der Arretierungsvorrichtung (60) in einer ersten Position zumindest einen Teil eines der Hohlräume (62) zur Aufnahme des Verschlusses (56) bildet, so dass der Verschluss (56) dieser Kartusche (52) in dieser ersten Position in den Hohlraum (62) versenkbar ist und in einer zweiten, gedrehten Position der Arretierungsvorrichtung (60) nicht in den Hohlraum (62) versenkbar ist.

13. Kartuschensystem nach Anspruch 12, **dadurch gekennzeichnet, dass**
ein Austragsrohr (66) an der Arretierungsvorrichtung (60) angeordnet ist und über eine Durchführung durch die Arretierungsvorrichtung (60) mit dem Mischraum in der ersten Position verbunden ist, wobei vorzugsweise die Auslassöffnung des Mischraums durch die Arretierungsvorrichtung (60) in der zweiten Position verschlossen ist.

## Claims

1. A cartridge system for mixing and applying a mixing ware, particularly a medical cement, comprising at least two cartridges (2, 52) arranged parallel to each other, **characterized by**
a mixing space (18) comprising an outlet opening, whereby the cartridges (2, 52) each comprise cartridge walls with at least one opening (16, 64) that connect the cartridges (2, 52) to the mixing space (18), the cartridges (2, 52) each comprise a feed plunger (8, 58) for expelling starting components (4, 54) of the mixing ware from the cartridges (2, 52) through the openings (16, 64) and at least one closure (6, 56) is assigned to each cartridge (2, 52), the at least one closure (6, 56) is shiftable in longitudinal direction of the cartridges (2, 52), wherein these closures (6, 56) close the at least one openings (16, 64) of the cartridges (2, 52) in a starting position and the at least one openings (16, 64) are at least partially open in an end position of the closures (6, 56), wherein at least two of the of the cartridges (2, 52) comprise a cylindrical hollow space (20, 62) and the closures (6, 56) are arranged inside the cartridges (2, 52), wherein the closures (6, 56) are cylindrical in shape and are lowerable into the cylindrical hollow spaces (20, 62) in the end position for exposing the openings (16, 64).

2. The cartridge system according to claim 1, wherein the cylindrical hollow spaces (20, 62) of the cartridges (2, 52) are connected to the surrounding through at least one ventilation opening (24, 68), such that upon lowering the closure (6, 56) air escapes from the hollow space (20, 62), whereby preferably the hollow spaces (20, 62) are located in the front part of the cartridge system and the ventilation openings (24, 68) are located in cartridge heads (22) of the cartridges (2, 52).

3. The cartridge system according to claim 1 or 2, wherein the closures (6, 56) separate the hollow spaces (20, 62) from the cartridges (2, 52) filled with the starting components (4, 54), preferably separate tightly, especially preferably separate fluidtight and/or gastight.

4. The cartridge system according to any one of the preceding claims, wherein the shiftable closures (6, 56) are arranged in the cartridges (2, 52) in a press-fit.

5. The cartridge system according to any one of the preceding claims, wherein the closures (6, 56) are compact bodies or hollow bodies, preferably hollow cylinders having at least one closed base surface.

6. The cartridge system according to any one of the preceding claims, wherein the hollow spaces (20, 62) have an inner diameter that is equal to or larger than an external diameter of the corresponding closure (6, 56), and/or the hollow spaces (20, 62) are of a length of at least one tenth of the height of the closure (6, 56).

7. The cartridge system according to any one of the preceding claims, wherein at least one blocking organ is arranged in each cartridge (2, 52) which limits the axial movement of the closure (6, 56) above the outlet opening.

8. The cartridge system according to any one of the preceding claims, wherein the feed plunger (8, 58) in each cartridge (2, 52) is connected in a force-fitting manner to the closure (6, 56) of said cartridge (2, 52) by the starting component (4, 54) arranged in the cartridge (2, 52) such that a force acting on the feed plungers (8, 58) is transferable to the closures (6, 56) and the closures (6, 56) are transferable from the starting position to the end position.

9. The cartridge system according to any one of the preceding claims, wherein the feed plungers (8, 58) and closures (6, 56) are arranged in pairs, each in a tube (34), which each contain one starting component (4, 54) in between, wherein the closed tubes (34) are arranged in the cartridges (2, 52), especially behind the openings (16, 64) if viewed from the cartridge head (22).

10. The cartridge system according to any one of the preceding claims, wherein an optical indicator (32) is arranged on at least one of the closures (6, 56), allowing to visualize the starting position and differentiating the starting position from the end position by at least one inspection opening (24, 68) and/or inspection window (68) in at least one cartridge (2, 52), especially in at least one of the cartridge heads (22), and/or which projects through said at least one inspection opening (24, 68) when the closure (6, 56) is in the end position, whereby the inspection opening (24, 68) preferably also serves as the ventilation opening (24, 68).

11. The cartridge system according to any one of the preceding claims, wherein the mixing space (18) is solidly or detachably connected to a dispensing tube (26, 66) including a static mixer (28), whereby preferably the static mixer (28) projects into the mixing space (18).

12. The cartridge system according to any one of the preceding claims, wherein a rotatable locking device (60) is supported in a bearing in a bracket in the region of the cartridge heads such as to be rotatable, wherein the locking device (60) is a rotationally symmetrical body having at least one cylindrical recess (62), preferably having a recess for each cartridge (52), wherein the locking device (60) at least in areas forms at least one cartridge head (22) and the recess (62) of the locking device (60), in a first position, forms at least one part of one of the hollow spaces (62) for accommodating the closure (56) such that the closure (56) of said cartridge (52) is lowerable into the hollow space (62) in this first position and is not lowerable into the hollow space (62) in a second, rotated position of the locking device (60).

13. The cartridge system according to claim 12, wherein a dispensing tube (66) is arranged on the locking device (60) and, in the first position, is connected to the mixing space by a conduct through the locking device (60), wherein preferably, in the second position, the outlet opening of the mixing space is closed by the locking device (60).

## Revendications

1. Système de cartouches pour le mélange et l'application d'un matériau mixte, notamment, un ciment médical, comprenant au moins deux cartouches (2, 52) disposées parallèles l'une à l'autre, **caractérisé par**
une chambre de mélange (18) avec un orifice de sortie, où les cartouches (2, 52) comprennent respectivement au moins un orifice (16, 64) dans les parois de cartouches, qui relient les cartouches (2, 52) avec la chambre de mélange (18), les cartouches (2, 52) comprennent respectivement un piston de transport (8, 58) pour l'expulsion de composants de départ (4, 54) du matériau mixte hors des cartouches (2, 52) par les orifices (16, 64), et à chaque cartouche (2, 52) est associé au moins un système de fermeture (6, 56) pouvant coulisser dans la direction longitudinale des cartouches (2, 52), où les systèmes de fermeture (6, 56) ferment les orifices (16, 64) des cartouches (2, 52) dans une position de départ et les orifices (16, 64) sont ouverts au moins par endroits dans une position finale des systèmes de fermeture (6, 56) pouvant coulisser, et au moins deux cartouches (2, 52) comprennent respectivement un espace creux (20, 62) cylindrique et les systèmes de fermeture (6, 56) sont disposés à l'intérieur des cartouches (2, 52), où les systèmes de fermeture (6, 56) ont une forme cylindrique et peuvent être enfouis dans leur position finale dans les espaces creux (20, 62) cylindriques des cartouches (2, 52) afin de libérer les orifices (16, 64).

2. Système de cartouches selon la revendication 1, **caractérisé en ce que**
les espaces creux (20, 62) cylindriques des cartouches (2, 52) sont reliés avec l'environnement par l'intermédiaire d'au moins un orifice de mise à l'air (24, 68) de sorte que, lors de l'enfouissement du système de fermeture (6, 56), l'air s'échappe de l'espace creux (20, 62), où, de préférence, les espaces creux (20, 62) sont de préférence disposés dans la région frontale du système de cartouches et les orifices de mise à l'air (24, 68) sont disposés dans les têtes de cartouches (22) des cartouches (2, 52).

3. Système de cartouches selon la revendication 1 ou 2, **caractérisé en ce que**
les systèmes de fermeture (6, 56) séparent les espaces creux (20, 62) avec les régions des cartouches (2, 52) remplies avec les composants de départ (4, 54), de préférence, les séparent de manière étanche, de manière particulièrement préférée, les séparent de manière étanche aux fluides et/ou aux gaz.

4. Système de cartouches selon l'une des revendications précédentes, **caractérisé en ce que**
les systèmes de fermeture (6, 56) pouvant coulisser sont disposés par un emboîtement dans les cartouches (2, 52).

5. Système de cartouches selon l'une des revendications précédentes, **caractérisé en ce que**
les systèmes de fermeture (6, 56) sont des corps compacts ou des corps creux, notamment, des cylindres creux avec au moins une surface de base fermée.

6. Système de cartouches selon l'une des revendications précédentes, **caractérisé en ce que**
les espaces creux (20, 62) possèdent un diamètre intérieur qui est égal ou supérieur au diamètre extérieur du système de fermeture (6, 56) correspondant, et/ou qui ont une longueur d'au moins un dixième de la hauteur du système de fermeture (6, 56).

7. Système de cartouches selon l'une des revendications précédentes, **caractérisé en ce que**,
dans chaque cartouche (2, 52), au moins un organe de blocage est disposé qui limite le déplacement axial du système de fermeture (6, 56) au dessus de l'orifice de sortie.

8. Système de cartouches selon l'une des revendications précédentes, **caractérisé en ce que**,
dans chaque cartouche (2, 52), le piston de transport (8, 58) est relié par complémentarité des matières avec le système de fermeture (6, 56) de cette cartouche (2, 52) par l'intermédiaire du composant de départ (2, 52) disposé dans la cartouche (2, 52), de sorte que, sous l'action d'une force sur les pistons de transport (8, 58), la force est transmise sur les systèmes de fermeture (6, 56) et les systèmes de fermeture (6, 56) peuvent être passés de la position de départ vers la position finale.

9. Système de cartouches selon l'une des revendications précédentes, **caractérisé en ce que**
les pistons de transport (8, 58) et les systèmes de fermeture (6, 56) sont disposés respectivement par paires dans un tube (34) qui contient entre ceux-ci respectivement un composant de départ (4, 54), où les tubes (34) fermés sont disposés dans les cartouches (2, 52), notamment, vu à partir de la tête de cartouche (22), derrière les orifices (16, 64).

10. Système de cartouches selon l'une des revendications précédentes, **caractérisé en ce**
**qu'**au niveau d'au moins un système de fermeture (6, 56), un indicateur optique (32) est disposé, par lequel la position de départ peut être différenciée visuellement de la position finale par au moins un orifice de visualisation (24, 63), et/ou une fenêtre de visualisation (68), dans au moins une cartouche (2, 52), notamment par au moins un orifice de visualisation (24, 68), et/ou une fenêtre de visualisation (68), dans au moins une des têtes de cartouche (22), et/ou qui dépasse hors de la cartouche (2, 52) par cet au moins un orifice de visualisation (24, 68) lorsque le système de fermeture (6, 56) se trouve dans la position finale, où, de préférence, l'orifice de visualisation (24, 68) est également l'orifice de mise à l'air (24 68).

11. Système de cartouches selon l'une des revendications précédentes, **caractérisé en ce que**
la chambre de mélange (18) est reliée solidement ou de manière amovible avec un tube d'évacuation (26, 66) avec un mélangeur statique (28), où le mélangeur statique (28) s'étend de préférence dans la chambre de mélange (18).

12. Système de cartouches selon l'une des revendications précédentes, **caractérisé en ce**
**qu'**un dispositif de blocage (60) pouvant être mis en rotation est logé en pouvant tourner dans un support dans la région des têtes des cartouches, où le dispositif de blocage (60) est un corps symétrique en rotation avec au moins une cavité (62) cylindrique, de préférence, avec une cavité pour chaque cartouche (52), où le dispositif de blocage (60) forme au moins par endroits au moins une tête de cartouche (22) et la cavité (62) du dispositif de blocage (60) forme au moins une partie de l'un des espaces creux (62) pour la réception du système de fermeture (56) dans une première position, de sorte que le système de fermeture (56) de cette cartouche (52) peut être enfoui dans l'espace creux (62) dans cette première position et, dans une deuxième position tournée du dispositif de blocage (60), ne peut pas être enfoui dans l'espace creux (62).

13. Système de cartouches selon la revendication 12, **caractérisé en ce**
**qu'**un tube d'évacuation (66) est agencé sur le dispositif de blocage (60) et est relié avec la chambre de mélange par l'intermédiaire d'un passage à travers le dispositif de blocage (60) dans la première position, et de préférence l'orifice d'évacuation de la chambre de mélange est fermé par le dispositif de blocage (60) dans la deuxième position.
